# EUROPÄISCHE PATENTANMELDUNG

(11) **EP 0 717 964 A1**
(43) Veröffentlichungstag der Anmeldung: **26.06.1996**
(21) Anmeldenummer: 95119603.9
(22) Anmeldetag: 13.12.1995
(51) Int. Cl.: A61B 17/36

(54) **Laserchirurgischer Applikator**

(30) Priorität: 22.12.1994 DE 4445908
(71) Anmelder: Dornier Medizintechnik GmbH, D-82110 Germering (DE)
(72) Erfinder: Hauptmann, Gerhard, D-81827 München (DE); Rother, Werner, Dr., D-64331 Weiterstadt (DE)

(57) **Zusammenfassung**

Der laserchirurgischer Applikator dient zur Kontaktvaporisation von Gewebe und weist eine Lichtleitfaser mit Quarzglaskern auf, an deren distalem Ende eine Kontaktspitze aus einem optisch transparenten, festen Material angeordnet ist. Die Kontaktspitze weist eine Quarzglashülse auf, in welcher zumindest der Quarzglaskern der Lichtleitfaser mit einem Abstand zur Innenwand koaxial verläuft und wobei das distale Ende des Quarzglaskernes mit dem distalen Ende der Quarzglashülse zu einer optisch transparenten Spitze verschmolzen ist.

## Beschreibung

Die Erfindung betrifft einen laserchirurgischer Applikator zur Kontaktvaporisation von Gewebe mit einer Lichtleitfaser mit Quarzglaskern, an deren distalem Ende eine aus einer Quarzglashülse hergestellte Kontaktspitze angeordnet ist, wobei das distale Ende des Quarzglaskernes mit dem distalen Ende der Quarzglashülse zu einer optisch transparenten Spitze verschmolzen ist. (Die Begriffe distal bzw. proximal sind hier und im folgenden Text auf die Strahlungsquelle, also auf das Lasergerät bezogen).

Für die Vaporisation von Gewebe mittels Nd:YAG-Laserstrahlung sind am Gewebe hohe Leistungsdichten und somit eine hohe Laserleistung erforderlich. Viele Gewebearten weisen an ihrer Oberfläche zunächst einen spiegelnden Flüssigkeitsfilm auf, der die Laserstrahlung ablenkt, so daß sekundäre Schädigungen verursacht werden können. Aufgrund dessen besteht seit langem der Wunsch nach einer sog. Kontaktvaporisation, welche erstmals mittels sog. Saphirspitzen (z.B. US 4 693 244), die auf das Ende des die Laserstrahlung führenden Lichtleiters aufgesetzt waren, ermöglicht wurde. Derartige Applikatoren sind konstruktiv sehr aufwendig, da eine Kühlung der Verbindungsstelle zwischen Saphier und Lichtleiter erforderlich ist und sind zudem wegen des verwendeten Saphirs relativ teuer. Zudem besteht die Gefahr, daß bei Ausfall der Kühlung die Verbindung zwischen Saphir und Lichtleiter überhitzt wird und damit die Saphirspitze abfallen kann.

Aus der EP 0 514 258 A1 ist ein Applikator der o.g. Art bekannt, bei dem eine zylindrische Hülse das Ende einer Lichtleitfaser umgibt und mit dieser zu einer Kontaktspitze verschmolzen ist. Durch die enge Passung zwischen der Hülse und der Lichtleitfaser ist der Bereich der Verschmelzung nicht exakt einstellbar, so daß auch der Bereich, aus dem das Laserlicht die Kontaktspitze verläßt, nicht vorherbestimmbar ist. Außerdem tritt das Laserlicht stark divergent aus der Kontaktspitze aus, insbesondere bei Verwendung von Diodenlasern.

Es ist daher Aufgabe der vorliegenden Erfindung, einen laserchirurgischen Applikator zur Kontaktvaporisation von Gewebe zu schaffen, welcher konstruktiv einfach und billig herzustellen ist, den erhöhten Sicherheitsanforderungen in der Laserchirurgie genügt und eine verbesserte Konvergenz des abgestrahlten Laserlichtes, insbesondere bei Verwendung von Diodenlasern ermöglicht. Die Lösung dieser Aufgabe gelingt durch einen Applikator gemäß Patentanspruch 1.

Aus der WO 92/17243 ist eine optisch transparente Hohlnadel aus Kunststoff bekannt, in der das freie Ende einer Lichtleitfaser mündet, welche in Gewebe einstechbar ist. Dieser Applikator dient jedoch zur Hyperthermie und ist aufgrund der verwendeten Materialien (Kunststoff) für die Vaporisation nicht verwendbar. Eine lichtdurchlässige Kappe, welche über das abgemantelte Ende eines Lichtleiters aufgeschoben ist, ist auch in der DE 39 19 322 C2 beschrieben, wobei diese Sonde ebenfalls nur zur Hyperthermie verwendbar ist und wobei auch bei dieser Sonde, wie schon im Falle der oben erwähnten WO 92/17243 das Laserlicht innerhalb der Kappe aus dem Lichtleiter austritt und zuerst durch die Wandungen der Kappe hindurchtreten muß.

Im Gegensatz zu den aufgesetzten Spitzen nach dem bekannten Stand der Technik gibt es zwischen Lichtleiter und Spitze keinen die Laserstrahlung schwächenden Luftspalt mehr, welcher vom Licht überwunden werden muß, bevor dieses die Kontaktspitze verläßt. Durch den trichterförmigen Übergang vom Lichtleiter zur Kontaktspitze wird erreicht, daß Lichtstrahlen, die mit großer Apertur im Lichtleiter verlaufen gebündelt werden und insbesondere bei konvex geformten Kontaktspitzen konvergent aus diesen austreten. Dadurch wird die Laserstrahlung fokussiert und eine hohe Leistungsdichte am Applikator-Gewebe-Übergang erzielt. Diese Leistungsdichte führt punktuell zu einer schnellen Erhitzung des Gewebes und somit zur raschen Entstehung eines Karbonisationskeimes. Unmittelbar nach Entstehung dieses Karbonisationskeimes wird die folgende Laserstrahlung von dieser Karbonisationsschicht stark absorbiert, so daß der Karbonisationsvorgang großflächig aufrechterhalten wird.

Durch den Verschmelzungsprozeß ist zudem eine mechanisch feste Verbindung zwischen der Kontaktspitze und dem Lichtleiter gegeben, wobei eine Zentrierung des letzteren lediglich während des Herstellungsprozesses notwendig ist. Sollte die Mantelschicht (Buffer) des Lichtleiters ebenfalls aus Quarzglas bestehen, so kann diese bei der Verschmelzung mit der Quarzglashülse einbezogen werden.

Zur Anpassung an verschiedene anatomische Verhältnisse und Veränderung der Abstrahlcharakteristik kann der aufgeschmolzene Bereich auch keil- und/oder kegelförmig ausgebildet sein, wobei diese Bereiche stets nur so groß ausgeführt werden, daß sie noch von dem aus der Lichtleitfaser heraus divergierenden Licht erfaßt werden.

Die Erfindung wird im folgenden anhand der in den Figuren teilweise schematisch dargestellten Ausführungsbeispiele näher beschrieben. Es zeigen:
- Fig. 1: einen Querschnitt durch einen Applikator mit Kontaktspitze
- Fig. 1a: einen Querschnitt durch einen Teilbereich des Applikators gemäß Fig. 1
- Fig. 2: einen Querschnitt durch einen Applikator mit meiselförmiger Kontaktspitze
- Fig. 3: einen Querschnitt durch einen Applikator mit kegelförmiger Kontaktspitze
- Fig. 4: einen Querschnitt durch einen Applikator mit seitlich abgeflachter Kontaktspitze.

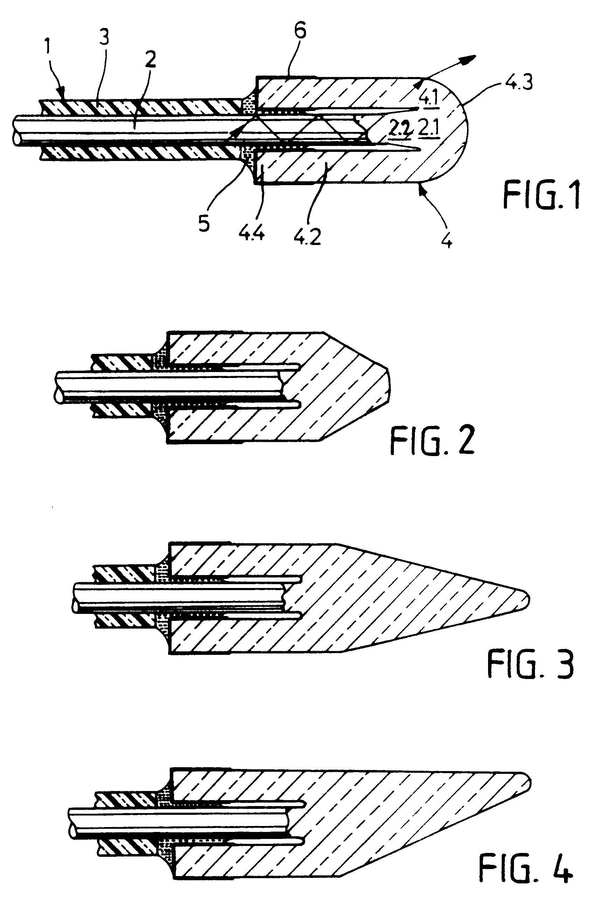

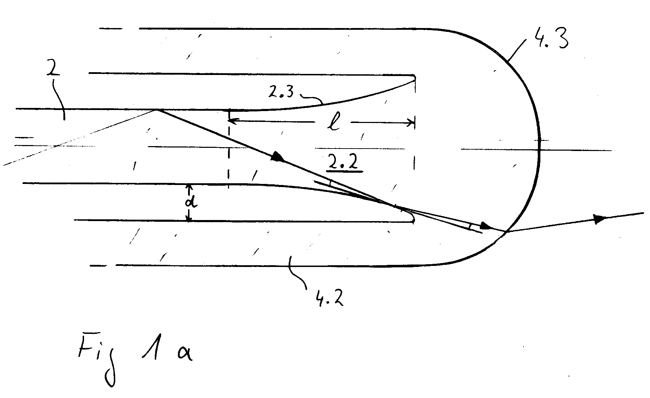

Der in den Fig. 1 bzw. 1a dargestellte Applikator weist eine Lichtleitfaser 1 mit Quarzglaskern 2 und Kunststoffummantelung 3 auf, wobei letztere am Ende im Bereich von 1 bis 2 cm vom Quarzglaskern 2 entfernt ist. Die Kontaktspitze 4 besteht aus einer Quarzglashülse 4.2, welche den Kern 2 des Lichtleiters 1 pilzförmig umgibt und mit diesem in dem Bereich 2.1 bzw. 4.1 verschmolzen ist derart, daß eine optisch transparente Spitze 4.3 mit halbkugelförmiger Oberfläche gebildet wird, aus welcher die Laserstrahlung austritt. Zwischen der Innenwand der Hülse 4.2 und der Mantelfläche des Faserkerns 2 besteht ein umlaufend möglichst gleicher Abstand d, der durch einen trichterförmigen Übergangsbereich 2.2 überbrückt wird. Der optimale Abstand d bestimmt sich letztlich aus der Länge l und dem Maß der Erweiterung des Trichters. Bei einem Trichter mit von Innen nach Außen zunehmendem Öffnungswinkel, bestimmt sich dessen optimale Länge l durch den Punkt, in dem ein Lichtstrahl mit maximaler Apertur den Trichtermantel tangential berührt. In Fig. 1a ist ein Lichtstrahl nahe der Aperturgrenze schematisch dargestellt. Ein derartiger Lichtstrahl trifft im Bereich des Trichters 2.2 auf dessen gekrümmte Mantelfläche und wird von dort in Richtung auf die optische Achse des Lichtleiters 2 reflektiert. In Verbindung mit einer konvexen Austrittsfläche der Spitze 4.3 findet eine weitere konvergierende Wirkung statt.

Zur Herstellung einer Kontaktspitze gemäß Fig. 1a wird eine zylindrische Quarzglashülse verwendet, durch welche das abgemantelte Ende eines Lichtleiterkerns 2 hindurchgesteckt wird. Falls die Ummantelung 3 des Lichtleiters 1 ebenfalls aus Quarzglas besteht, so ist ein Abmanteln nicht erforderlich. Das aus der Hülse herausragende Ende des Lichtleiters wird aufgeschmolzen, derart, daß sich eine tropfenförmige Verdickung bildet, deren Durchmesser mindestens dem Innendurchmesser der hülse entspricht, vorteilhafterweise jedoch größer ist. Zwischen der Verdickung und dem restlichen Lichtleiter bildet sich so ein trichterförmiger Übergang 2.2. Die distalen Enden 2.1 und 4.1 des Lichtleiters 1 bzw. der Quarzglashülse 4 werden dann kurz bis zum Erweichungspunkt erhitzt, wobei diese miteinander verschmelzen und einen homogenen Glaskörper bilden. Der trichterförmige Übergang 2.2 muß bei dem letzten Schmelzvorgang erhalten bleiben. Für einen in der Laserchirurgie typischen Lichtleiterdurchmesser von 600µm mit einer numerischen Apertur von 0,37 (entspricht ca. 42° Vollwinkel) beträgt die optimale Länge l des Trichters etwa 3 mm.

Das distale Ende 4.4 der Quarzglashülse 4.2 wird gegenüber dem Lichtleiter 1 mit einem Klebekragen 5 verschlossen, so daß mit der Kontaktspitze auch in einem vollständig in einer Flüssigkeit eingetauchten Zustand gearbeitet werden kann. Der Kleber weist dabei einen niedrigeren Berechungsindex als der Kern 2 der Lichtleitfaser 1 auf, so daß in diesem Bereich des Kernes 2 noch Totalreflexion für die Laserstrahlung garantiert ist.

Da es nicht vollständig vermeidbar ist, daß Laserstrahlung von der Oberfläche 4.3 zurück in die Hülse 4 reflektiert wird, können an dem proximalen Applikatorende 4.4 noch vergleichsweise hohe Temperaturen auftreten. Deshalb kann es vorteilhaft sein, diesen Bereich durch eine Spiegelschicht 6 vor Strahlung zu schützen; hierfür eignen sich an sich bekannte Metall- oder λ/4-beschichtungen.

In den Figuren 2, 3 und 4 sind verschiedene Ausführungsformen von Kontaktspitzen dargestellt, wobei diejenige gemäß Fig. 2 im Bereich der Spitze meiselförmig ausgebildet ist und zur Behandlung von Hohlorganen dient. Die kegelförmig spitz zulaufende Kontaktspitze gemäß Figur 3 dient zur punktuellen Vaporisierung; diejenige gemäß Figur 4 mit einer seitlich abgeflachten Spitze wirkt einseitig und dient somit zur einseitigen Vaporisierung in Hohlorganen.

## Patentansprüche

1. Laserchirurgischer Applikator zur Kontaktvaporisation von Gewebe mit einer Lichtleitfaser mit Quarzglaskern, an deren distalem Ende eine aus einer Quarzglashülse hergestellte Kontaktspitze angeordnet ist, wobei das distale Ende des Quarzglaskernes mit dem distalen Ende der Quarzglashülse zu einer optisch transparenten Spitze (4.3) verschmolzen ist **dadurch gekennzeichnet,** daß der Quarzglaskern (2) der Lichtleitfaser (1) mit einem Abstand zur Innenwand der Quarzglashülse (4.2) koaxial verläuft und, daß der Übergang des Quarzglaskernes (2) zur Quarzglashülse (4.2) als sich erweiternder Trichter (2.2) ausgebildet ist.

2. Applikator nach Anspruch 1, **dadurch gekennzeichnet,** daß die Mantelfläche des Trichters (2.2) in Richtung auf das breitere Ende eine stetig zunehmende Krümmung aufweist.

3. Applikator nach Anspruch 1 oder 2 , **dadurch gekennzeichnet,** daß die optisch transparente Spitze (4.3) halbkugelförmig ausgebildet ist.

4. Applikator nach Anspruch 1 oder 2 , **dadurch gekennzeichnet,** daß die optisch transparente Spitze keil- und/oder kegelförmig ausgebildet ist (Fig. 2 bis 4).

5. Applikator nach einem der Ansprüche 1 bis 4, **dadurch gekennzeichnet,** daß das proximale Ende (4.4) der Quarzglashülse (4.2) mit der Lichtleiterfaser (1) verklebt ist.

6. Applikator nach Anspruch 5, **dadurch gekennzeichnet,** daß der Brechungsindex des Klebstoffes (5) niedriger ist, als der des Faserkernes (2) .

7. Applikator nach einem der Ansprüche 1 bis 6, **dadurch gekennzeichnet,** daß das proximale Ende (4.4) der Quarzglashülse (4.2) zumindest im Bereich der Verklebung mit der Lichtleitfaser (1) eine strahlungsreflektierende Schicht (6) aufweist.

8. Applikator nach einem der Ansprüche 1 bis 7, **dadurch gekennzeichnet,** daß das Laserlicht aus der gesamten Oberfläche der optisch transparenten Spitze (4.3) austritt.

9. Applikator nach einem der Ansprüche 1 bis 8, **dadurch gekennzeichnet,** daß die Länge des nicht mit dem Quarzglaskern (2) der Lichtleitfaser (1) verschmolzenen Teils der Quarzglashülse (4.2) mindestens 10 mm beträgt.

10. Verfahren zur Herstellung eines Applikators nach einem der Ansprüche 1 bis 9, **gekennzeichnet** durch folgende Schritte
a) Aufschmelzen des distalen Endes einer Lichtleitfaser zur Bildung einer tropfenförmigen Verdickung mit trichterförmigem Übergang zur Lichtleitfaser,
b) Verschmelzen der Stirnfläche einer über die Lichtleitfaser geschobenen zylindrischen Hülse mit der tropfenförmigen Verdickung der Lichtleitfaser unter Beibehaltung des trichterförmigen Überganges.
